(19) Europäisches Patentamt / European Patent Office / Office européen des brevets

(11) **EP 4 442 196 B1**

(12) **EUROPEAN PATENT SPECIFICATION**

(45) Date of publication and mention
of the grant of the patent:
**08.04.2026 Bulletin 2026/15**

(51) International Patent Classification (IPC):
$A61B\ 5/02^{(2006.01)}$  $G06T\ 7/00^{(2017.01)}$
$G06T\ 7/60^{(2017.01)}$  $G06V\ 10/82^{(2022.01)}$
$G16H\ 30/40^{(2018.01)}$  $G16H\ 50/20^{(2018.01)}$
$G16H\ 50/70^{(2018.01)}$  $G06T\ 7/11^{(2017.01)}$
$G06V\ 10/26^{(2022.01)}$  $G06V\ 10/25^{(2022.01)}$
$G06V\ 10/44^{(2022.01)}$  $A61B\ 5/00^{(2006.01)}$
$A61B\ 5/021^{(2006.01)}$

(21) Application number: 23213400.7

(22) Date of filing: **30.11.2023**

(52) Cooperative Patent Classification (CPC):
G16H 30/40; A61B 5/004; A61B 5/02007;
A61B 5/021; A61B 5/4244; A61B 5/7267;
G06T 7/0012; G06T 7/11; G06T 7/60; G06V 10/25;
G06V 10/26; G06V 10/454; G06V 10/82;
G16H 50/20; G16H 50/70;                    (Cont.)

(54) **CIRRHOTIC PORTAL HYPERTENSION DIAGNOSING METHOD, APPARATUS, DEVICE, AND MEDIUM**

VERFAHREN, VORRICHTUNG UND MEDIUM ZUR DIAGNOSE VON ZIRRHOTISCHER PORTALHYPERTONIE

PROCÉDÉ DE DIAGNOSTIC DE L'HYPERTENSION PORTALE CIRRHOTIQUE, APPAREIL, DISPOSITIF ET SUPPORT

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**

(30) Priority: **07.04.2023 CN 202310373801**

(43) Date of publication of application:
**09.10.2024 Bulletin 2024/41**

(73) Proprietor: **Qi, Xiaolong**
**210000 Nanjing (CN)**

(72) Inventors:
• **QI, Xiaolong**
  **Nanjing 210000 (CN)**
• **WANG, Chengyan**
  **Shanghai 201203 (CN)**
• **HUANG, Yifei**
  **Shanghai 201203 (CN)**

(74) Representative: **Witthoff Jaekel Steinecke Patentanwälte PartG mbB**
**Widdersdorfer Straße 236-240**
**50825 Köln (DE)**

(56) References cited:
**US-A1- 2019 117 094**

• **WANG KEXIN ET AL**: "Non-invasive Assessment of Hepatic Venous Pressure Gradient (HVPG) Based on MR Flow Imaging and Computational Fluid Dynamics", 21 September 2021, 20210921, PAGE(S) 33 - 42, XP047622227

• **GEORGE STEPHANIE M ET AL**: "Hemodynamics in Normal and Diseased Livers: Application of Image-Based Computational Models", CARDIOVASCULAR ENGINEERING AND TECHNOLOGY, SPRINGER INTERNATIONAL PUBLISHING, CHAM, vol. 6, no. 1, 23 December 2014 (2014-12-23), pages 80 - 91, XP035454396, ISSN: 1869-408X, [retrieved on 20141223], DOI: 10.1007/S13239-014-0195-5

• **DANA J⬦R⬦MY ET AL**: "Conventional and artificial intelligence-based imaging for biomarker discovery in chronic liver disease", HEPATOLOGY INTERNATIONAL, SPRINGER INDIA, INDIA, vol. 16, no. 3, 9 February 2022 (2022-02-09), pages 509 - 522, XP037888159, ISSN: 1936-0533, [retrieved on 20220209], DOI: 10.1007/S12072-022-10303-0

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- **YIKUN WANG ET AL: "MRI-based liver vascular morphology and surface nodularity quantification for assessment of portal hypertension", PROCEEDINGS OF THE JOINT ANNUAL MEETING ISMRM-ESMRMB 2022 & ISMRT ANNUAL MEETING, LONDON, UK, 07-12 MAY 2022, ISMRM, 2030 ADDISON STREET, 7TH FLOOR, BERKELEY, CA 94704 USA, no. 1527, 22 April 2022 (2022-04-22), XP040728075**

(52) Cooperative Patent Classification (CPC): (Cont.)
G06T 2207/10081; G06T 2207/10088;
G06T 2207/20072; G06T 2207/20081;
G06T 2207/20084; G06T 2207/30056;
G06T 2207/30101; G06T 2207/30176;
G06V 2201/031; Y02A 90/10

## Description

## Technical Field

The present disclosure relates to the technical field of medical diagnosis, and in particular, to a cirrhotic portal hypertension diagnosing method, an apparatus, a device, and a medium.

## Background Art

Portal hypertension non-invasive detection methods include serological examination, anatomical image marker, and physical substitution method based on histological characteristics. Currently, there still lacks a non-invasive gold standard that can replace HVPG for portal hypertension risk stratification and monitoring therapeutic effects. The main problem thereof lies in that invasive detection of portal hypertension is restricted in clinical applications, while the non-invasive risk stratification method of portal hypertension still needs to be further studied and improved: the evaluation efficacy is insufficient, and the invasive gold standard cannot be replaced; and there is insufficient generalization performance for different causes and races.

Although the technique proposed in the prior art for evaluating portal hypertension based on radiomics has achieved certain breakthroughs in evaluation efficacy, this technique is limited in research errors due to different causes, populations, and heterogeneity of examination machines, and inaccurate results caused by model establishment based on a two-dimensional plane and insufficiently optimized calculation parameters. Wang et al. "Non-invasive Assessment of Hepatic Venous Pressure Gradient (HVPG) Based on MR Flow Imaging and Computational Fluid Dynamics" (Fluid Dynamics, 21 September 2021, pages 33-42) discloses that clinically significant portal hypertension (CSPH) is a severe complication of chronic liver disease associated with cirrhosis, which is diagnosed by the measurement of hepatic venous pressure gradient (HVPG). However, HVPG measurement is invasive and therefore difficult to be widely applied in clinical routines. There is no currently available technique to measure HVPG noninvasively. Computational fluid dynamics (CFD) has been used for noninvasive measurement of vascular pressure gradient in the intracranial and coronary arteries. However, it has been scarcely employed in the hepatic vessel system due to the difficulties in reconstructing precise vascular anatomies and setting appropriate boundary conditions. Several computer tomography and ultrasound-based studies have verified the effectiveness of virtual HVPG (vHVPG) by directly connecting the portal veins and hepatic veins before CFD simulations. We apply the latest techniques of phase-contrast magnetic resonance imaging (PC-MRI) and DIXON to obtain the velocity and vessel anatomies at the same time. Besides, we improve the CFD pipeline with regard to the construction of vessel connections and reduction of calculation time. The proposed method shows high accuracy in the CSPH diagnosis in a study containing ten healthy volunteers and five patients. The MRI-based noninvasive HVPG measurement is promising in the clinical application of CSPH diagnosis.

## Summary

In view of this, the present disclosure aims at providing a cirrhotic portal hypertension diagnosing apparatus and medium, which can realize precise non-invasive diagnosis of portal hypertension.

Described herein, but not part of the present invention, is also a cirrhotic portal hypertension diagnosing method, wherein the method includes steps of:

processing an input MRI image or CT image using a trained liver vessel three-dimensional segmentation model, so as to obtain a liver contour image of a patient, wherein the liver contour image includes a portal vascular tree, a hepatic vein vascular tree, an aortic vascular tree, and an inferior vena cava vascular tree;

extracting automatically corresponding vascular geometric parameters from each vascular tree in the obtained liver contour image; and

processing input vascular geometric parameters using a trained cirrhotic portal hypertension diagnostic model, so as to obtain a cirrhotic portal hypertension diagnosis result of the patient.

An embodiment of the present disclosure provides a cirrhotic portal hypertension diagnosing apparatus, wherein the apparatus includes:

a processing module, configured to process an input MRI image or CT image using a trained liver vessel three-dimensional segmentation model, so as to obtain a liver contour image of a patient, wherein the liver contour image includes a portal vascular tree, a hepatic vein vascular tree, an aortic vascular tree, and an inferior vena cava vascular tree;

a calculating module, configured to automatically extract corresponding vascular geometric parameters from each vascular tree in the obtained liver contour image; and

a diagnosing module, configured to process input vascular geometric parameters using a trained cirrhotic portal hypertension diagnostic model, so as to obtain a cirrhotic portal hypertension diagnosis result of the patient.

The liver vessel three-dimensional segmentation model adopts a U-Net network segmentation architecture, and before extracting features from the input MRI image or CT image, the liver vessel three-dimensional segmentation model further performs pre-processing, including steps of:

performing field-of-view cropping on the input MRI image or CT image according to a pre-set dimension;

performing resolution re-sampling on the MRI image or CT image having undergone the field-of-view cropping; and

slicing the re-sampled MRI image or CT image by means of trilinear interpolation, and normalizing signal strength using z-score, so as to obtain a pre-processed enhanced image.

In some embodiments, a coding path of the U-Net in the liver vessel three-dimensional segmentation model contains 5~20 convolution layers and pooling layers, each layer contains a $3\times3$ convolution kernel and a rectified linear unit activation function, and a $3\times3$ convolution layer with a stride of 2 is connected immediately after a first convolution layer.

In some embodiments, for the CT image, before a pre-processing pipeline is applied, a signal intensity window is set to be [-200,200]HU.

In some embodiments, the vascular geometric parameters include one or more of vessel volume; vessel volume percentage; the number of vessel branch nodes; the number of vessel terminal nodes; the number of vessel branches; whole vessel length; vessel main branch length; vessel sub-branch length; vessel main branch curvature; vessel sub-branch curvature; vessel main branch tortuosity; vessel sub-branch tortuosity; equivalent diameter, minimum diameter, and roundness of vessel main branch; and first sub-branch angle.

In some embodiments, the step of extracting automatically corresponding vascular geometric parameters from each vascular tree in the obtained liver contour image includes steps of:

identifying a portal vascular tree, a hepatic vein vascular tree, an aortic vascular tree, and an inferior vena cava vascular tree from the obtained liver contour image;

sampling central lines of the portal vascular tree, the hepatic vein vascular tree, the aortic vascular tree, and the inferior vena cava vascular tree, respectively; and

calculating corresponding vascular geometric parameters based on the central lines of the portal vascular tree, the hepatic vein vascular tree, the aortic vascular tree, and the inferior vena cava vascular tree.

In some embodiments, vessel tortuosity is calculated as follows: counting each branch of the central line of vessel, calculating an Euclidean distance and a curve distance thereof, and dividing the curve distance by the Euclidean distance and subtracting 1, so that the tortuosity of the branch is obtained.

In some embodiments, a support vector machine for binary classification is used, and an output cirrhotic portal hypertension diagnosis result is HVPG normal or HVPG abnormal.

In some embodiments, a pre-constructed cirrhotic portal hypertension diagnostic model is trained in a manner as follows, including steps of:

obtaining several sets of vascular geometric parameters as a data set using a trained liver vessel three-dimensional segmentation model;

labelling the data set with a true cirrhotic portal hypertension diagnosis result, and dividing the data set into a training set, a verification set, and a test set according to a set ratio;

training the pre-constructed cirrhotic portal hypertension diagnostic model based on the training set, and performing parameter adjustment on the pre-constructed cirrhotic portal hypertension diagnostic model based on the verification set, until parameters of the cirrhotic portal hypertension diagnostic model converge, so as to obtain a to-be-tested cirrhotic portal hypertension diagnostic model; and

evaluating the to-be-tested cirrhotic portal hypertension diagnostic model based on the test set, wherein if an evaluation result reaches a set threshold, the to-be-tested cirrhotic portal hypertension diagnostic model is determined as the trained cirrhotic portal hypertension diagnostic model.

In some embodiments, a pre-set ratio of the training set, the verification set, and the test set is 6:2:2.

In some embodiments, an Adam optimizer is selected to train the pre-constructed liver vessel three-dimensional segmentation model.

In some embodiments, an initial learning rate is set to be 0.0001, a batch size is set to be 4, an echo number is 100, and U-Net learns a consistent pattern from a training set composed of reference images with annotations, and then predicts images in all remaining cases.

An embodiment of the present disclosure provides a computer-readable storage medium, wherein the computer-readable storage medium stores a computer program, and the computer program, when run by a processor, executes the steps run by the cirrhotic portal hypertension diagnosing apparatus according to any one of the above.

The cirrhotic portal hypertension diagnosing apparatus, and medium in the present disclosure process the input MRI image or CT image using the trained liver vessel three-dimensional segmentation model, so as to obtain the liver contour image of the patient, wherein the liver contour image includes the portal vascular tree, the hepatic vein vascular tree, the aortic vascular tree, and the inferior vena cava vascular tree; automatically extract corresponding vascular geometric parameters from each vascular tree in the obtained liver contour image; process the input vascular geometric parameters using the trained cirrhotic portal hypertension diagnostic model, so as to obtain the cirrhotic portal hypertension diagnosis result of the patient. Thus, the cirrhotic portal hypertension is diagnosed through the vascular geometric characteristics, so that on one hand, the diagnosis result is more accurate, and on the other hand, powerful pathophysiological explanations can be supplemented to a model result.

## Brief Description of Drawings

In order to illustrate the technical solutions of embodiments of the present disclosure which relate to the cirrhotic portal hypertension diagnosing apparatus and the computer-readable storage medium more clearly, drawings that need to be used in the embodiments are introduced briefly below, and it should be understood that the following drawings merely show some embodiments of the present disclosure and a person ordinarily skilled in the art still could obtain other relevant drawings according to these drawings without using any creative efforts.

FIG. 1 shows a flowchart of a cirrhotic portal hypertension diagnosing method;

FIG. 2 shows a schematic diagram of processing an input MRI image or CT image using a trained liver vessel three-dimensional segmentation model in an embodiment of the present disclosure;

FIG. 3 shows a schematic diagram of extracting automatically corresponding vascular geometric parameters from each vascular tree in the obtained liver contour image in an embodiment of the present disclosure;

FIG. 4 shows a schematic diagram of obtaining a cirrhotic portal hypertension diagnosis result of a patient using a trained cirrhotic portal hypertension diagnostic model in an embodiment of the present disclosure;

FIG 5 shows a structural block diagram of a cirrhotic portal hypertension diagnosing apparatus provided in an embodiment of the present disclosure; and

FIG. 6 shows a structural block diagram of an electronic device.

## Detailed Description of Embodiments

In order to make objectives, technical solutions, and advantages of the embodiments of the present disclosure clearer, the technical solutions in the embodiments of the present disclosure will be described clearly and completely below in conjunction with drawings in the embodiments of the present disclosure. It should be understood that the drawings in the present disclosure are merely for the illustrative and descriptive purpose. Besides, it should be understood that the schematic drawings are not drawn to scale. **The** flowcharts used in the present disclosure show operations implemented according to some embodiments of the present disclosure. **It** should be understood that the operations of the flowcharts may be implemented out of order, and steps without contextual logic may be reversed in order or simultaneously implemented. **In** addition, one skilled in the art, guided by the present disclosure, could add one or more other operations to the flowcharts, or remove one or more operations from the flowcharts.

Besides, some but not all embodiments of the present disclosure are described. Generally, components in the embodiments of the present disclosure, as described and shown in the drawings herein, may be arranged and designed in various different configurations. Therefore, the detailed description below of the embodiments of the present disclosure provided in the drawings is not intended to limit the claimed scope of the present disclosure, but merely illustrates chosen embodiments of the present disclosure.

**It** should be noted that the term "include" will be used in the embodiments of the present disclosure to indicate the existence of features specified thereafter, but not to exclude addition of other features.

**In** view of the technical problem proposed in the background art, the present disclosure provides cirrhotic portal hypertension diagnosing apparatus, and medium, which can realize precise and non-invasive diagnosis of portal hypertension.

**In** order to facilitate understanding the present embodiment, the cirrhotic portal hypertension diagnosing apparatus, and medium provided in the embodiments of the present disclosure are introduced in detail below. Furthermore, the above-mentioned method is described in detail, which can be applied to a terminal device, and also can be applied to a server, wherein when the above method is applied to a server, it may be cloud medical treatment, i.e., a man-machine interaction interface is provided via a terminal device, the terminal device sends an operation instruction triggered by a user via a man-machine interaction screen to the server, and the server performs data processing in response to the user's operation instruction and returns a post-processing result (vascular geometric parameters and cirrhotic portal hypertension diagnosis) to the terminal device.

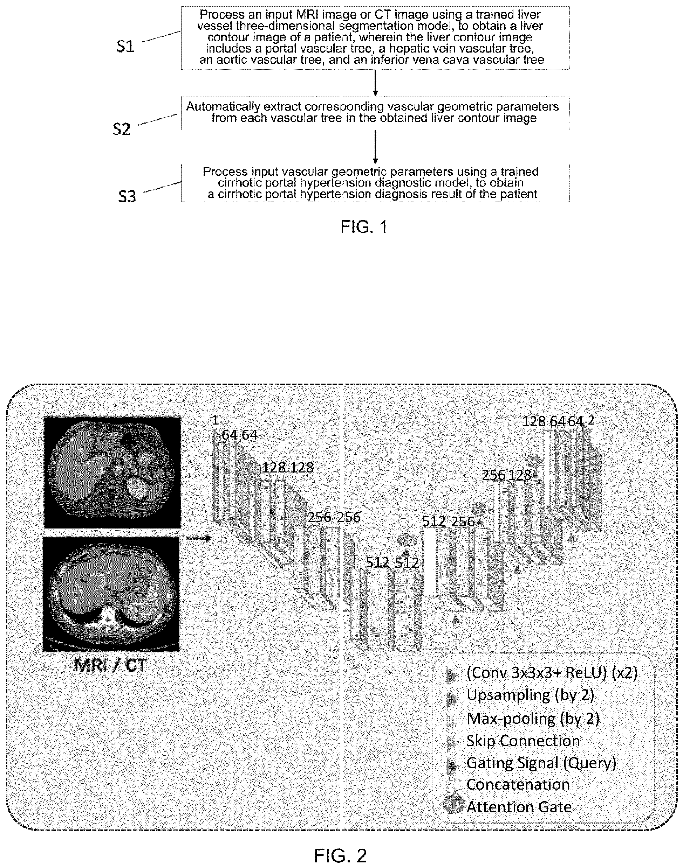

Referring to FIG. 1 of the description, a cirrhotic portal hypertension diagnosing method is described, wherein the method includes the following steps:

S1, processing an input **MRI** image or **CT** image using a trained liver vessel three-dimensional segmentation model, so as to obtain a liver contour image of a patient, wherein the liver contour image includes a portal vascular tree, a hepatic vein vascular tree, an aortic vascular tree, and an inferior vena cava vascular tree.

With reference to FIG. 2, in step S1, the liver vessel three-dimensional segmentation model adopts a U-Net network segmentation architecture, and before extracting features from the input MRI image or CT image, the liver vessel three-dimensional segmentation model further performs pre-processing, specifically including steps of: performing field-of-view cropping on the input MRI image or CT image according to a pre-set dimension; performing resolution re-sampling on the MRI image or CT image having undergone the field-of-view cropping; and slicing the re-sampled MRI image or CT image by means of trilinear interpolation, and normalizing signal strength using z-score, so as to obtain a pre-processed enhanced image.

A coding path of the U-Net in the liver vessel three-dimensional segmentation model may contain 5~20 convolution layers and pooling layers, each layer contains a $3\times3$ convolution kernel and a rectified linear unit (ReLu) activation function, and a $3\times3$ convolution layer with a stride of 2 is connected immediately after a first convolution layer. In the above, a joint feature is provided for two tasks by using a connection between two paths, so as to improve prediction performance. When pre-processing the patient's MRI image or CT image, the field of view is first cropped into $640\times640$ mm$^2$; image resampling resolution is $0.625\times0.625$ mm$^2$, and a size of a corresponding matrix is $512\times512$; a slice thickness is interpolated to 0.625 mm by means of trilinear interpolation; the signal intensity is normalized using the z-score, so as to obtain the enhanced image, particularly for a CT image, before a pre-processing pipeline is applied, a signal intensity window is set to be [-200,200]HU. Furthermore, the acquired enhanced image is input into the trained liver vessel three-dimensional segmentation model to extract the feature, thus obtaining the patient's liver contour image. In this embodiment, the obtained liver contour image includes the portal vascular tree, the hepatic vein vascular tree, the aortic vascular tree, and the inferior vena cava vascular tree.

It should be noted that, when training the pre-constructed liver vessel three-dimensional segmentation model, by selecting a DICE similarity coefficient as a loss function, a network can be effectively trained. In an embodiment, an Adam optimizer is selected to perform network training, wherein an initial learning rate is set to be 0.0001, a batch size is set to be 4, an echo number is 100, and U-Net learns a consistent pattern from a training set composed of reference images with annotations, and then predicts images in all remaining cases. Training time of the model is about 15 h, and testing time of each case is within 10 s. In the above, the process of training the pre-constructed liver vessel three-dimensional segmentation model should be a technical means well known to a person skilled in the art, and will not be repeated herein.

S2, extracting automatically corresponding vascular geometric parameters from each vascular tree in the obtained liver contour image.

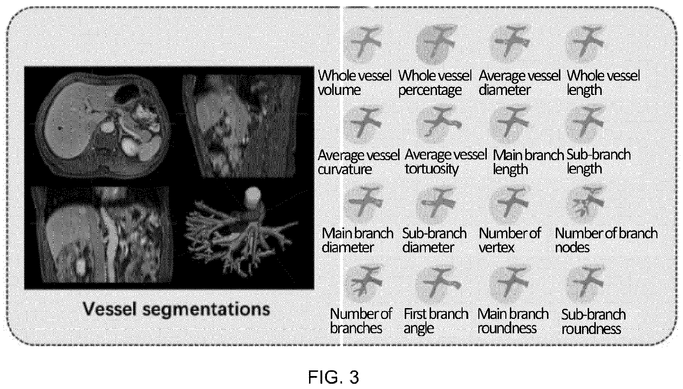

With reference to FIG. 3 of the description, in step S2, when calculating the vascular geometric parameters, first, the portal vascular tree, the hepatic vein vascular tree, the aortic vascular tree, and the inferior vena cava vascular tree, i.e. branch levels of vessel, should be identified from the liver contour image acquired in step S1. For example, a central line is extracted from a segmented vessel based on a decision-tree method, a graph representation of vascular system is constructed according to the extracted central line, and further automatic identification is performed according to features of the central lines of different vascular trees. **In** the present disclosure, the vascular geometric parameters include sixteen parameters in total, namely, vessel volume; vessel volume percentage; number of vessel branch nodes; number of vessel terminal nodes; number of vessel branches; whole vessel length; vessel main branch length; vessel sub-branch length; vessel main branch curvature; vessel sub-branch curvature; vessel main branch tortuosity; vessel sub-branch tortuosity; equivalent diameter, minimum diameter, and roundness of vessel main branch; and first sub-branch angle. With these sixteen parameters, powerful pathophysiological explanations can be provided for subsequent cirrhotic portal hypertension diagnosis, and the diagnostic accuracy can be improved.

When calculating the vascular geometric parameters, the central line can be appropriately sampled, and then parameters are calculated, so as to improve accuracy of the parameters and avoid influence of extreme values. Specifically:

$$\text{vessel volume (ml)} = \text{voxel\_count} \cdot \text{unit\_vol} \quad (volume = voxel\_count \times unit\_vol),$$

where voxel_count is the total number of pixel values greater than zero in the image, and unit_vol=spacing[0]*spacing[1]*spacing[2]*0.001 (*unit_vol=spacing*[0]*\*spacing*[1]*\*spacing*[2]*\*0.001*);

$$\text{vessel volume percentage} = \text{intrahepatic vessel volume} / \text{liver volume};$$

the number of vessel terminal nodes (terminal_node_num): traversing the central lines of the vessel, recording coordinates of a starting point and an ending point of each central line, and counting the number after getting rid of the same coordinates, so as to obtain the number of terminal nodes;

the number of vessel branch nodes (branch_node_num): traversing the central lines of the vessel, recording coordinates of bifurcations of the central lines, and counting the number of bifurcations;

the number of vessel branches (branch_num):

$$\text{branch\_num} = \text{terminal\_node\_num} + \text{branch\_node\_num} - 1;$$

vessel sub-branch length (branch_length): calculating length of the central line of each sub-branch;

vessel main branch length (main_length): identifying the central line corresponding to a main branch, and calculating the length;

whole vessel length (whole_length): summing lengths of all vessel branches;

vessel curvature calculation: the central line of vessel and a point p thereon, a circle being tangent to the central line at the point p, the circle being an osculating circle, and reciprocal of radius of the osculating circle being curvature of the point p;

vessel main branch curvature: counting each point in the central line of the vessel main branch, and calculating the curvatures and taking a mean value;

vessel sub-branch curvature: counting each point in the central line of the vessel sub-branch, and calculating the curvatures and taking a mean value;

calculation of vessel tortuosity: counting each branch of the central line of vessel, calculating an Euclidean distance (Euclid_dist) and a curve distance (curve_dist) thereof, and dividing the curve distance by the Euclidean distance and subtracting 1, so as to obtain the tortuosity of the branch (Tortuosity);

$$Tortuosity = \frac{euclid\_dist}{curve\_dist} - 1$$

vessel main branch tortuosity: calculating an Euclidean distance and a curve distance of the central line of the vessel main branch, and calculating the tortuosity;

vessel sub-branch tortuosity: calculating an Euclidean distance and a curve distance of the central line of each vessel sub-branch, and calculating the tortuosity and taking a mean value;

equivalent diameter, minimum diameter, and roundness of vessel main branch: a cross section of a point corresponding to the vessel needs to be used for calculating this parameter, where a normal vector $p_0p_1$ of a plane is formed by two adjacent points Po, P in the central line, and then a cross section area "area" and a cross section perimeter "perimeter" of the vessel of this plane at this point are calculated;

then, the equivalent diameter is:

$$e\_radius = 2 * \sqrt{\frac{area}{\pi}};$$

the minimum diameter is min_radius=diameter of maximum inscribed circle of a cross section at this point;

the roundness is:

$$roundness = 4 * \tau * \frac{area}{perimeter};$$

then the roundness of this cross section is calculated, where area is area of the cross section, and perimeter is perimeter of the cross section;

the first sub-branch angle: finding a sub-branch node $P_0$ at a vessel main branch, and midpoints $P_1$, $P_2$, $P_3$...of adjacent sub-branches, then respectively calculating an included angle $\theta$ of vectors $P_0P_1$ and $P_0P_2$, and so on, and finally, calculating a mean value of $\theta_0$, $\theta_1$...

$$\theta_i = arccos\left(\frac{P_0P_{i+1} \cdot P_0P_{i+1}}{\|P_0P_{i+1}\| \cdot \|P_0P_{i-1}\|}\right)$$

$$\theta = \frac{\sum_{i=1}^{n} \theta_i}{n}$$

S3, processing the input vascular geometric parameters using a trained cirrhotic portal hypertension diagnostic model, so as to obtain a cirrhotic portal hypertension diagnosis result of the patient.

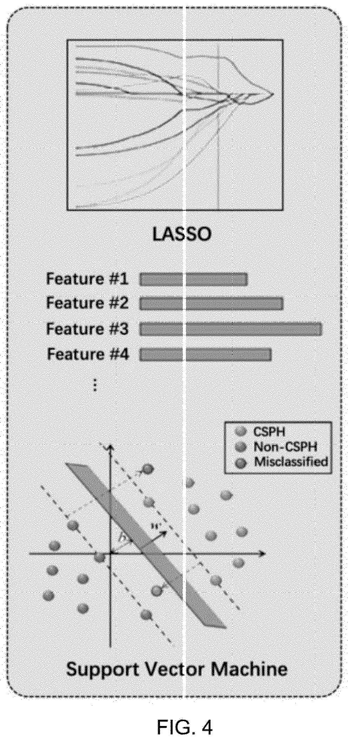

With reference to FIG. 4 of the description, in step S3, the cirrhotic portal hypertension diagnostic model uses a support vector machine for binary classification, and uses a non-linear kernel function, so as to output a cirrhotic portal hypertension diagnosis result of **HVPG** normal or **HVPG** abnormal according to the input vascular geometric parameters.

**In** the above, when training the pre-constructed cirrhotic portal hypertension diagnostic model, the trained liver vessel three-dimensional segmentation model is used to obtain several sets of vascular geometric parameters as a data set; the data set is labeled with a true cirrhotic portal hypertension diagnosis result, and the data set is divided into a training set, a verification set, and a test set according to a set ratio; in an embodiment, a pre-set ratio of the training set, the verification set, and the test set is 6:2:2, then general parameters, such as weight and bias, of the pre-constructed cirrhotic portal hypertension diagnostic model are trained based on the training set, and performing parameter adjustment on the pre-constructed cirrhotic portal hypertension diagnostic model based on the verification set, for example, learning rate and the number of network layers are adjusted, until parameters of the cirrhotic portal hypertension diagnostic model converge, so as to obtain a to-be-tested cirrhotic portal hypertension diagnostic model; finally, the to-be-tested cirrhotic portal hypertension diagnostic model is evaluated based on the test set, wherein if an evaluation result reaches a set threshold, for example, the set threshold is an accuracy rate of 0.9, the to-be-tested cirrhotic portal hypertension diagnostic model is determined as the trained cirrhotic portal hypertension diagnostic model. **In** the above, the process of training the pre-constructed cirrhotic portal hypertension diagnostic model should be a technical means well known to a person skilled in the art, and will not be repeated here.

Instead of directly inputting the liver contour image output by the liver vessel three-dimensional segmentation model into the cirrhotic portal hypertension diagnostic model to acquire the cirrhotic portal hypertension diagnosis result of the patient, the cirrhotic portal hypertension diagnosing firstly processes the liver contour image, and extract the vascular geometric parameters from each vascular tree in the liver contour image, then inputs the vascular geometric parameters into the cirrhotic portal hypertension diagnostic model, so as to acquire the cirrhotic portal hypertension diagnosis result of the patient, so that on one hand, the diagnosis result is more accurate, and on the other hand, powerful pathophysiological explanations can be supplemented to the diagnosis result. **In** addition, the liver vessel three-dimensional segmentation model used is based on an **MRI/CT** multi-modal image, which is beneficial to clinical application and popularization.

Based on the above-described concept, an embodiment of the present disclosure provides a cirrhotic portal hypertension diagnosing apparatus, and as the principle for solving the problem by the apparatus in the embodiment of the present disclosure is similar to that of the cirrhotic portal hypertension diagnosing method, reference can be made to implementation of the method for implementation of the apparatus, and repetition will not be made herein.

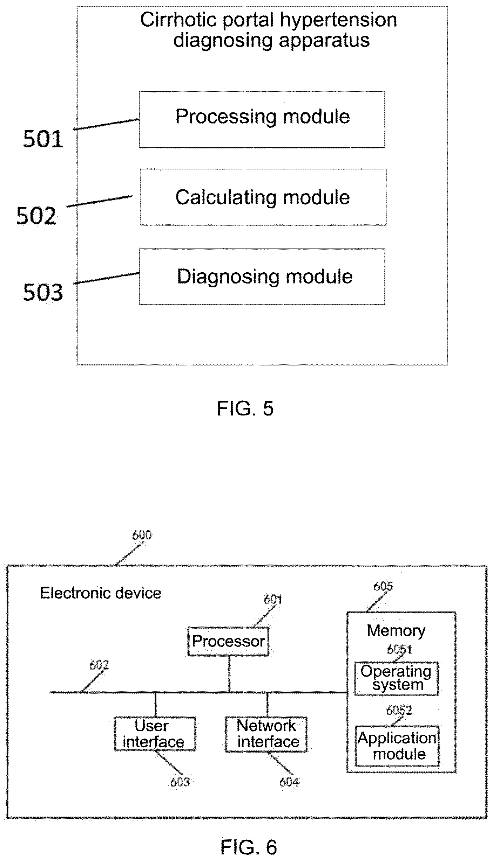

As shown in FIG. 5 of the description, the present disclosure provides a cirrhotic portal hypertension diagnosing apparatus, wherein the apparatus includes:

a processing module 501, configured to process an input **MRI** image or **CT** image using a trained liver vessel three-dimensional segmentation model, so as to obtain a liver contour image of a patient, wherein the liver contour image includes a portal vascular tree, a hepatic vein vascular tree, an aortic vascular tree, and an inferior vena cava vascular tree;

a calculating module 502, configured to automatically extract corresponding vascular geometric parameters from each vascular tree in the obtained liver contour image; and

a diagnosing module 503, configured to process input vascular geometric parameters using a trained cirrhotic portal hypertension diagnostic model, so as to obtain a cirrhotic portal hypertension diagnosis result of the patient.

**The** liver vessel three-dimensional segmentation model adopts a u-net network segmentation architecture, and the processing module 501 further performs pre-processing on features of the **MRI** image or **CT** image input into the trained liver vessel three-dimensional segmentation model, including:

performing field-of-view cropping on the input MRI image or CT image according to a pre-set dimension;

performing resolution re-sampling on the MRI image or CT image having undergone the field-of-view cropping; and

slicing the re-sampled MRI image or CT image by means of trilinear interpolation, and normalizing signal strength using z-score, so as to obtain a pre-processed enhanced image.

In some embodiments, extracting automatically corresponding vascular geometric parameters from each vascular tree in the obtained liver contour image by the calculating module 502 includes:

identifying a portal vascular tree, a hepatic vein vascular tree, an aortic vascular tree, and an inferior vena cava vascular tree from the obtained liver contour image;

sampling central lines of the portal vascular tree, the hepatic vein vascular tree, the aortic vascular tree, and the inferior vena cava vascular tree, respectively; and

calculating corresponding vascular geometric parameters based on the central lines of the portal vascular tree, the hepatic vein vascular tree, the aortic vascular tree, and the inferior vena cava vascular tree.

In some embodiments, the diagnosing module 503 further trains a pre-constructed cirrhotic portal hypertension diagnostic model, including:

obtaining several sets of vascular geometric parameters as a data set using a trained liver vessel three-dimensional segmentation model;

labelling the data set with a true cirrhotic portal hypertension diagnosis result, and dividing the data set into a training set, a verification set, and a test set according to a set ratio;

training the pre-constructed cirrhotic portal hypertension diagnostic model based on the training set, and performing parameter adjustment on the pre-constructed cirrhotic portal hypertension diagnostic model based on the verification set, until parameters of the cirrhotic portal hypertension diagnostic model converge, so as to obtain a to-be-tested cirrhotic portal hypertension diagnostic model; and

evaluating the to-be-tested cirrhotic portal hypertension diagnostic model based on the test set, wherein if an evaluation result reaches a set threshold, the to-be-tested cirrhotic portal hypertension diagnostic model is determined as the trained cirrhotic portal hypertension diagnostic model.

The cirrhotic portal hypertension diagnosing apparatus provided in the present disclosure processes the input MRI image or CT image using the trained liver vessel three-dimensional segmentation model through the processing module, so as to obtain the liver contour image of the patient, wherein the liver contour image includes the portal vascular tree, the hepatic vein vascular tree, the aortic vascular tree, and the inferior vena cava vascular tree; automatically extracts corresponding vascular geometric parameters from each vascular tree in the obtained liver contour image through the calculating module; process the input vascular geometric parameters using the trained cirrhotic portal hypertension diagnostic model through the diagnosing module, so as to obtain the cirrhotic portal hypertension diagnosis result of the patient. Thus, the cirrhotic portal hypertension is diagnosed through the vascular geometric characteristics, so that on one hand, the diagnosis result is more accurate, and on the other hand, powerful pathophysiological explanations can be supplemented to a model result.

Based on the same concept of the present disclosure, as shown in FIG. 6 of the description, a structure of an electronic device 600 is described, but not part of the present invention, the electronic device 600 includes: at least one processor 601, at least one network interface 604 or other user interfaces 603, a memory 605, and at least one communication bus 602. The communication bus 602 is configured to enable connection communication between these components. This electronic device 600 optionally includes the user interface 603, including a display (for example, a touchscreen, an LCD, a CRT, a holographic imager or a projector), a keyboard, or a click device (for example, a mouse, a trackball, a touch panel, or a touchscreen).

The memory 605 may include a read-only memory and a random access memory, and provides an instruction and data to the processor 601. A part of the memory 605 may also include a non-volatile random access memory (NVRAM).

The memory 605 may store the following elements: a protectable module or a data structure, or a subset thereof, or an extension set thereof:

an operating system 6051, containing various system programs, configured to realize various basic services and process hardware-based tasks; and

an application module 6052, containing various applications, such as a launcher, a media player, and a browser, configured to realize various application services.

By invoking a program or an instruction stored in the memory 605, the processor 601 may be configured for executing steps in the cirrhotic portal hypertension diagnosing method, and can realize precise and non-invasive diagnosis of portal hypertension.

The present disclosure further provides a computer-readable storage medium, wherein the computer-readable storage medium stores a computer program, and the computer program, when run by a processor, executes the steps in the cirrhotic portal hypertension diagnosing method.

Specifically, the storage medium can be a general-purpose storage medium, for example, removable disk and hard disk, and when the computer program on the storage medium is run, the above cirrhotic portal hypertension diagnosing method can be executed.

In the embodiments provided in the present disclosure, it should be understood that the apparatus and the method disclosed may be implemented in other manners. The apparatus embodiment described in the above is merely exemplary, for example, the units are merely divided according to logical functions, but they may be divided in other manners in practical implementation, for another example, multiple units or components may be combined or may be integrated into another system, or some features may be omitted, or not executed. In addition, mutual couplings or direct coupling or communication connection as shown or discussed may be indirect coupling or communication connection via some communication interfaces, means or units, and may be in an electrical form, a mechanical form or other forms.

The units described as separate parts may be or also may not be physically separated, the parts displayed as units may be or also may not be physical units, i.e., they may be located at one place, or also may be distributed on a plurality of network units. Some or all of the units may be selected according to actual needs to achieve the purpose of the solution in the present embodiment.

Besides, various functional units in the embodiments of the present disclosure may be integrated into one processing unit, or each unit also may exist in a physically independent way, or two or more than two units may be integrated into one unit.

**If** a function is realized in a form of software functional unit and is sold or used as an independent product, it may be stored in a computer readable storage medium. Based on such understanding, the technical solutions of the present disclosure in essence or parts making contribution to the prior art or parts of the technical solutions can be embodied in form of a software product, and this computer software product is stored in a storage medium, including several instructions for making one computer device (which can be a personal computer, a server or a network device etc.) execute all or part of the steps of the methods of various embodiments of the present disclosure. **The** aforementioned storage medium includes various media in which program codes can be stored, such as U disk, mobile hard disk, read-only memory (ROM), random access memory (RAM), magnetic disk or optical disk.

Finally, it should be indicated that the above embodiments are merely specific implementations of the present disclosure, for illustrating the technical solutions of the present disclosure.

## Claims

1.  A cirrhotic portal hypertension diagnosing apparatus comprising:

    a processing module, configured to process an input MRI image or CT image using a trained liver vessel three-dimensional segmentation model, so as to obtain a liver contour image of a patient, wherein the liver contour image comprises a portal vascular tree, a hepatic vein vascular tree, an aortic vascular tree, and an inferior vena cava vascular tree;
    a calculating module, configured to automatically extract corresponding vascular geometric parameters from each vascular tree in the obtained liver contour image; and
    a diagnosing module, configured to process input vascular geometric parameters using a trained cirrhotic portal hypertension diagnostic model, so as to obtain a cirrhotic portal hypertension diagnosis result of the patient; wherein
    the liver vessel three-dimensional segmentation model adopts a U-Net network segmentation architecture, and before extracting features from the input MRI image or CT image, the liver vessel three-dimensional segmentation model further performs pre-processing, comprising steps of:

    performing field-of-view cropping on the input MRI image or CT image according to a pre-set dimension;
    performing resolution re-sampling on the MRI image or CT image having undergone the field-of-view cropping; and
    slicing the re-sampled MRI image or CT image by means of trilinear interpolation, and normalizing signal strength using z-score, so as to obtain a pre-processed enhanced image.

2.  The cirrhotic portal hypertension diagnosing apparatus according to claim 1, wherein a coding path of the U-Net in the liver vessel three-dimensional segmentation model contains 5~20 convolution layers and pooling layers, each layer contains a $3\times3$ convolution kernel and a rectified linear unit activation function, and a $3\times3$ convolution layer with a stride of 2 is connected immediately after a first convolution layer.

3.  The cirrhotic portal hypertension diagnosing apparatus according to claim 1 or 2, wherein for the CT image, before a pre-processing pipeline is applied, a signal intensity window is set to be [-200,200]HU.

4.  The cirrhotic portal hypertension diagnosing apparatus according to any one of claims 1 to 3, wherein the vascular geometric parameters comprise one or more of the group consisting of vessel volume; vessel volume percentage; number of vessel branch nodes; number of vessel terminal nodes; number of vessel branches; whole vessel length; vessel main branch length; vessel sub-branch length; vessel main branch curvature; vessel sub-branch curvature; vessel main branch tortuosity; vessel sub-branch tortuosity; equivalent diameter, minimum diameter, and roundness of vessel main branch; and first sub-branch angle.

5.  The cirrhotic portal hypertension diagnosing apparatus according to any one of claims 1 to 4, wherein the step of extracting automatically corresponding vascular geometric parameters from each vascular tree in the obtained liver contour image comprises steps of:

identifying a portal vascular tree, a hepatic vein vascular tree, an aortic vascular tree, and an inferior vena cava vascular tree from the obtained liver contour image;

sampling central lines of the portal vascular tree, the hepatic vein vascular tree, the aortic vascular tree, and the inferior vena cava vascular tree, respectively; and

calculating corresponding vascular geometric parameters based on the central lines of the portal vascular tree, the hepatic vein vascular tree, the aortic vascular tree, and the inferior vena cava vascular tree.

6. The cirrhotic portal hypertension diagnosing apparatus according to claim 5, wherein vessel tortuosity is calculated as follows: counting each branch of the central line of vessel, calculating an Euclidean distance and a curve distance thereof, and dividing the curve distance by the Euclidean distance and subtracting 1, so that the tortuosity of the branch is obtained.

7. The cirrhotic portal hypertension diagnosing apparatus according to any one of claims 1 to 6, wherein a support vector machine for binary classification is used, and an output cirrhotic portal hypertension diagnosis result is HVPG normal or HVPG abnormal.

8. The cirrhotic portal hypertension diagnosing apparatus according to any one of claims 1 to 7, wherein a pre-constructed cirrhotic portal hypertension diagnostic model is trained in a manner as follows, comprising steps of:

obtaining several sets of vascular geometric parameters as a data set using a trained liver vessel three-dimensional segmentation model;

labelling the data set with a true cirrhotic portal hypertension diagnosis result, and dividing the data set into a training set, a verification set, and a test set according to a set ratio;

training the pre-constructed cirrhotic portal hypertension diagnostic model based on the training set, and performing parameter adjustment on the pre-constructed cirrhotic portal hypertension diagnostic model based on the verification set, until parameters of the cirrhotic portal hypertension diagnostic model converge, so as to obtain a to-be-tested cirrhotic portal hypertension diagnostic model; and

evaluating the to-be-tested cirrhotic portal hypertension diagnostic model based on the test set, wherein if an evaluation result reaches a set threshold, the to-be-tested cirrhotic portal hypertension diagnostic model is determined as the trained cirrhotic portal hypertension diagnostic model.

9. The cirrhotic portal hypertension diagnosing apparatus according to claim 8, wherein a pre-set ratio of the training set, the verification set, and the test set is 6:2:2.

10. The cirrhotic portal hypertension diagnosing apparatus according to claim 8 or 9, wherein an Adam optimizer is selected to train the pre-constructed liver vessel three-dimensional segmentation model.

11. The cirrhotic portal hypertension diagnosing apparatus according to claim 10, wherein an initial learning rate is set to be 0.0001, a batch size is set to be 4, an echo number is 100, and U-Net learns a consistent pattern from a training set composed of reference images with annotations, and then predicts images in all remaining cases.

12. A computer-readable storage medium, wherein the computer-readable storage medium stores a computer program, and the computer program, when run by a processor, executes the steps run by the cirrhotic portal hypertension diagnosing apparatus according to any one of claims 1 to 11.

**Patentansprüche**

1. Vorrichtung zur Diagnose einer zirrhotischen Portalhypertonie, umfassend:

ein Verarbeitungsmodul, das dazu eingerichtet ist, ein eingegebenes MRT-Bild oder CT-Bild unter Verwendung eines trainierten dreidimensionalen Segmentierungsmodells für Lebergefäße zu verarbeiten, um ein Leberkonturbild eines Patienten zu erhalten, wobei das Leberkonturbild einen Pfortader-Gefäßbaum, einen Lebervenen-Gefäßbaum, einen Aorten-Gefäßbaum und einen Gefäßbaum der unteren Hohlvene umfasst;

ein Berechnungsmodul, das dazu eingerichtet ist, automatisch entsprechende vaskuläre geometrische Parameter aus jedem Gefäßbaum in dem erhaltenen Leberkonturbild zu extrahieren; und

ein Diagnosemodul, das dazu eingerichtet ist, eingegebene vaskuläre geometrische Parameter unter Verwendung eines trainierten Diagnosemodells für zirrhotische Portalhypertonie zu verarbeiten, um ein Diagnoseergebnis hinsichtlich zirrhotischer Portalhypertonie des Patienten zu erhalten;

wobei das dreidimensionale Segmentierungsmodell für Lebergefäße eine U-Netzwerk-Segmentierungsarchitektur verwendet und vor der Merkmalsextraktion aus dem eingegebenen MRT-Bild oder CT-Bild ferner eine Vorverarbeitung durchführt, umfassend die Schritte:

Durchführen eines Sichtfeld-Zuschnitts des eingegebenen MRT-Bildes oder CT-Bildes gemäß einer vorgegebenen Dimension;
Durchführen einer Auflösungs-Neuabtastung des einem Sichtfeld-Zuschnitt unterzogenen MRT-Bildes oder CT-Bildes; und
Schneiden des neu abgetasteten MRT-Bildes oder CT-Bildes mittels trilinearer Interpolation und Normalisieren der Signalstärke unter Verwendung eines Z-Scores, um ein vorverarbeitetes, verbessertes Bild zu erhalten.

2. Vorrichtung zur Diagnose einer zirrhotischen Portalhypertonie nach Anspruch 1, wobei ein Kodierungspfad des U-Netzes im dreidimensionalen Segmentierungsmodell für Lebergefäße 5~20 Faltungs- und Pooling-Schichten enthält, wobei jede Schicht einen $3\times3$-Faltungskern und eine Rectified-Linear-Unit-Aktivierungsfunktion umfasst, und wobei unmittelbar nach einer ersten Faltungsschicht eine $3\times3$-Faltungsschicht mit einer Schrittweite von 2 angeschlossen ist.

3. Vorrichtung zur Diagnose einer zirrhotischen Portalhypertonie nach Anspruch 1 oder 2, wobei für das CT-Bild vor Anwendung einer Vorverarbeitungspipeline ein Signalintensitätsfenster auf [-200, 200] HU eingestellt wird.

4. Vorrichtung zur Diagnose einer zirrhotischen Portalhypertonie nach einem der Ansprüche 1 bis 3, wobei die vaskulären geometrischen Parameter eines oder mehrere aus der Gruppe, bestehend aus Gefäßvolumen; prozentualer Gefäßvolumenanteil; Anzahl der Gefäßverzweigungsknoten; Anzahl der Gefäßendknoten; Anzahl der Gefäßverzweigungen; Gesamtlänge der Gefäße; Länge der Hauptäste; Länge der Nebenäste; Krümmung der Hauptäste; Krümmung der Nebenäste; Tortuosität der Hauptäste; Tortuosität der Nebenäste; äquivalenter Durchmesser, Mindestdurchmesser und Rundheit der Hauptäste; sowie Winkel eines ersten Nebenastes, umfassen.

5. Vorrichtung zur Diagnose einer zirrhotischen Portalhypertonie nach einem der Ansprüche 1 bis 4, wobei der Schritt des automatischen Extrahierens entsprechender vaskulärer geometrischer Parameter aus jedem Gefäßbaum in dem erhaltenen Leberkonturbild folgende Schritte umfasst:

Identifizieren eines Pfortader-Gefäßbaums, eines Lebervenen-Gefäßbaums, eines Aorten-Gefäßbaums und eines Gefäßbaums der unteren Hohlvene aus dem erhaltenen Leberkonturbild;
Abtasten von Mittellinien des Pfortader-Gefäßbaums, des Lebervenen-Gefäßbaums, des Aorten-Gefäßbaums bzw. des Gefäßbaums der unteren Hohlvene; und
Berechnen entsprechender vaskulärer geometrischer Parameter basierend auf den Mittellinien des Pfortader-Gefäßbaums, des Lebervenen-Gefäßbaums, des Aorten-Gefäßbaums und des Gefäßbaums der unteren Hohlvene.

6. Vorrichtung zur Diagnose einer zirrhotischen Portalhypertonie nach Anspruch 5, wobei die Gefäßtortuosität wie folgt berechnet wird: Zählen jedes Astes der Mittellinie eines Gefäßes, Berechnen einer euklidischen Distanz und einer Kurvendistanz hierfür und Dividieren der Kurvendistanz durch die euklidische Distanz sowie Subtrahieren von 1, sodass die Tortuosität des Astes erhalten wird.

7. Vorrichtung zur Diagnose einer zirrhotischen Portalhypertonie nach einem der Ansprüche 1 bis 6, wobei eine Support-Vector-Maschine zur binären Klassifikation verwendet wird und das ausgegebene Diagnoseergebnis hinsichtlich zirrhotischer Portalhypertonie HVPG normal oder HVPG abnormal ist.

8. Vorrichtung zur Diagnose einer zirrhotischen Portalhypertonie nach einem der Ansprüche 1 bis 7, wobei ein vorab konstruiertes Diagnosemodell für zirrhotische Portalhypertonie wie folgt trainiert wird, umfassend die Schritte:

Erhalten mehrerer Sätze vaskulärer geometrischer Parameter als Datensatz unter Verwendung eines trainierten dreidimensionalen Segmentierungsmodells für Lebergefäße;
Kennzeichnen des Datensatzes mit einem tatsächlichen Diagnoseergebnis hinsichtlich zirrhotischer Portalhypertonie und Aufteilen des Datensatzes in einen Trainingssatz, einen Validierungssatz und einen Testsatz gemäß einem vorgegebenen Verhältnis;

Trainieren des vorab konstruierten Diagnosemodells für zirrhotische Portalhypertonie auf Basis des Trainingssatzes und Durchführen einer Parameteranpassung des vorab konstruierten Diagnosemodell für zirrhotische Portalhypertonie auf Basis des Validierungssatzes, bis die Parameter des Diagnosemodells für zirrhotische Portalhypertonie konvergieren, um ein zu testendes Diagnosemodell für zirrhotische Portalhypertonie zu erhalten; und

Bewerten des zu testenden Diagnosemodells für zirrhotische Portalhypertonie auf Basis des Testsatzes, wobei, wenn ein Bewertungsergebnis einen vorgegebenen Schwellenwert erreicht, das zu testende Diagnosemodell für zirrhotische Portalhypertonie als trainiertes Diagnosemodell für zirrhotische portale Hypertonie bestimmt wird.

9. Vorrichtung zur Diagnose einer zirrhotischen Portalhypertonie nach Anspruch 8, wobei das vorgegebene Verhältnis von Trainingssatz, Validierungssatz und Testsatz 6:2:2 beträgt.

10. Vorrichtung zur Diagnose einer zirrhotischen Portalhypertonie nach Anspruch 8 oder 9, wobei ein Adam-Optimierer zum Trainieren des vorab konstruierten dreidimensionalen Segmentierungsmodells für Lebergefäße ausgewählt wird.

11. Vorrichtung zur Diagnose einer zirrhotischen Portalhypertonie nach Anspruch 10, wobei eine anfängliche Lernrate auf 0,0001 eingestellt wird, eine Batch-Größe auf 4 eingestellt wird, eine Epochenzahl 100 beträgt und das U-Net ein konsistentes Muster aus einem Trainingssatz, der aus Referenzbildern mit Annotationen besteht, erlernt und anschließend Bilder in allen verbleibenden Fällen vorhersagt.

12. Computerlesbares Speichermedium, wobei das computerlesbare Speichermedium ein Computerprogramm speichert, und das Computerprogramm, wenn es von einem Prozessor ausgeführt wird, die von der Vorrichtung zur Diagnose einer zirrhotischen Portalhypertonie nach einem der Ansprüche 1 bis 11 ausgeführten Schritte ausführt.

**Revendications**

1. Appareil de diagnostic de l'hypertension portale cirrhotique comprenant :

un module de traitement, qui est configuré pour traiter une image IRM ou une image TDM en entrée à l'aide d'un modèle d'extraction tridimensionnelle segmentée des vaisseaux hépatiques entraîné, de façon à obtenir une image de contour hépatique d'un patient, laquelle image de contour hépatique comprend un arbre vasculaire portal, un arbre vasculaire de la veine hépatique, un arbre vasculaire aortique et un arbre vasculaire de la veine cave inférieure ;

un module de calcul, qui est configuré pour extraire automatiquement des paramètres géométriques vasculaires correspondants à partir de chaque arbre vasculaire dans l'image de contour hépatique obtenue ; et

un module de diagnostic, qui est configuré pour traiter des paramètres géométriques vasculaires en entrée à l'aide d'un modèle de diagnostic de l'hypertension portale cirrhotique entraîné, de façon à obtenir un résultat de diagnostic de l'hypertension portale cirrhotique du patient ;

lequel le modèle d'extraction tridimensionnelle segmentée des vaisseaux hépatiques adopte une architecture de segmentation en réseau U-Net, et avant d'extraire des caractéristiques à partir de l'image IRM ou de l'image TDM en entrée, le modèle d'extraction tridimensionnelle segmentée des vaisseaux hépatiques effectue en outre un prétraitement, comprenant les étapes consistant à :

effectuer un découpage du champ de vision sur l'image IRM ou l'image TDM en entrée selon une dimension prédéfinie ;

effectuer un rééchantillonnage de la résolution sur l'image IRM ou l'image TDM ayant subi le découpage du champ de vision ; et

trancher l'image IRM ou l'image TDM rééchantillonnée au moyen d'une interpolation trilinéaire, et normaliser l'intensité du signal à l'aide d'un score-z, de façon à obtenir une image améliorée prétraitée.

2. Appareil de diagnostic de l'hypertension portale cirrhotique selon la revendication 1, lequel un chemin d'encodage du U-Net dans le modèle d'extraction tridimensionnelle segmentée des vaisseaux hépatiques contient de 5 à 20 couches de convolution et couches de regroupement, chaque couche contenant un noyau de convolution $3\times3$ et une fonction

d'activation linéaire rectifiée, et une couche de convolution 3×3 avec un pas de 2 étant connectée immédiatement après une première couche de convolution.

3. Appareil de diagnostic de l'hypertension portale cirrhotique selon la revendication 1 ou 2, lequel pour l'image TDM, avant qu'une chaîne de prétraitement ne soit appliquée, une fenêtre d'intensité du signal est définie à [-200,200]HU.

4. Appareil de diagnostic de l'hypertension portale cirrhotique selon l'une quelconque des revendications 1 à 3, lequel les paramètres géométriques vasculaires comprennent un ou plusieurs éléments du groupe constitué par : volume vasculaire ; pourcentage de volume vasculaire ; nombre de nœuds de branche vasculaire ; nombre de nœuds terminaux vasculaires ; nombre de branches vasculaires ; longueur totale du vaisseau ; longueur de la branche principale du vaisseau ; longueur de la sous-branche du vaisseau ; courbure de la branche principale du vaisseau ; courbure de la sous-branche du vaisseau ; tortuosité de la branche principale du vaisseau ; tortuosité de la sous-branche du vaisseau ; diamètre équivalent, diamètre minimal et circularité de la branche principale du vaisseau ; et angle de la première sous-branche.

5. Appareil de diagnostic de l'hypertension portale cirrhotique selon l'une quelconque des revendications 1 à 4, lequel l'étape d'extraction automatique des paramètres géométriques vasculaires correspondants à partir de chaque arbre vasculaire dans l'image de contour hépatique obtenue comprend les étapes consistant à :

identifier un arbre vasculaire portal, un arbre vasculaire de la veine hépatique, un arbre vasculaire aortique et un arbre vasculaire de la veine cave inférieure à partir de l'image de contour hépatique obtenue ;
échantillonner les lignes centrales de l'arbre vasculaire portal, de l'arbre vasculaire de la veine hépatique, de l'arbre vasculaire aortique et de l'arbre vasculaire de la veine cave inférieure, respectivement ; et
calculer les paramètres géométriques vasculaires correspondants sur la base des lignes centrales de l'arbre vasculaire portal, de l'arbre vasculaire de la veine hépatique, de l'arbre vasculaire aortique et de l'arbre vasculaire de la veine cave inférieure.

6. Appareil de diagnostic de l'hypertension portale cirrhotique selon la revendication 5, lequel la tortuosité du vaisseau est calculée comme suit : compter chaque branche de la ligne centrale du vaisseau, calculer une distance euclidienne et une distance curviligne de celle-ci, et diviser la distance curviligne par la distance euclidienne puis soustraire 1, de sorte que la tortuosité de la branche soit obtenue.

7. Appareil de diagnostic de l'hypertension portale cirrhotique selon l'une quelconque des revendications 1 à 6, lequel une machine à vecteurs de support pour classification binaire est utilisée, et un résultat de diagnostic de l'hypertension portale cirrhotique en sortie est HVPG normal ou HVPG anormal.

8. Appareil de diagnostic de l'hypertension portale cirrhotique selon l'une quelconque des revendications 1 à 7, lequel un modèle de diagnostic de l'hypertension portale cirrhotique pré-construit est entraîné selon la méthode suivante, comprenant les étapes consistant à :

obtenir plusieurs ensembles de paramètres géométriques vasculaires en tant qu'ensemble de données à l'aide d'un modèle d'extraction tridimensionnelle segmentée des vaisseaux hépatiques entraîné ;
étiqueter l'ensemble de données avec un véritable résultat de diagnostic de l'hypertension portale cirrhotique, et diviser l'ensemble de données en un ensemble d'entraînement, un ensemble de validation et un ensemble de test selon un rapport prédéfini ;
entraîner le modèle de diagnostic de l'hypertension portale cirrhotique pré-construit sur la base de l'ensemble d'entraînement, et effectuer un ajustement des paramètres sur le modèle de diagnostic de l'hypertension portale cirrhotique pré-construit sur la base de l'ensemble de validation, jusqu'à ce que les paramètres du modèle de diagnostic de l'hypertension portale cirrhotique convergent, de façon à obtenir un modèle de diagnostic de l'hypertension portale cirrhotique à tester ; et
évaluer le modèle de diagnostic de l'hypertension portale cirrhotique à tester sur la base de l'ensemble de test, lequel si un résultat d'évaluation atteint un seuil défini, le modèle de diagnostic de l'hypertension portale cirrhotique à tester est déterminé comme étant le modèle de diagnostic de l'hypertension portale cirrhotique entraîné.

9. Appareil de diagnostic de l'hypertension portale cirrhotique selon la revendication 8, lequel un rapport prédéfini de l'ensemble d'entraînement, de l'ensemble de validation et de l'ensemble de test est de 6:2:2.

10. Appareil de diagnostic de l'hypertension portale cirrhotique selon la revendication 8 ou 9, lequel un optimiseur Adam est sélectionné pour entraîner le modèle d'extraction tridimensionnelle segmentée des vaisseaux hépatiques pré-construit.

11. Appareil de diagnostic de l'hypertension portale cirrhotique selon la revendication 10, lequel un taux d'apprentissage initial est défini à 0,0001, une taille de lot est définie à 4, un nombre d'époques est de 100, et U-Net apprend un schéma cohérent à partir d'un ensemble d'entraînement composé d'images de référence avec annotations, puis prédit les images dans tous les cas restants.

12. Support de stockage lisible par ordinateur, lequel le support de stockage lisible par ordinateur stocke un programme informatique, et le programme informatique, lorsqu'il est exécuté par un processeur, exécute les étapes exécutées par l'appareil de diagnostic de l'hypertension portale cirrhotique selon l'une quelconque des revendications 1 à 11.

FIG. 1

(Conv 3x3x3+ ReLU) (x2)
Upsampling (by 2)
Max-pooling (by 2)
Skip Connection
Gating Signal (Query)
Concatenation
Attention Gate

MRI / CT

FIG. 2

FIG. 3

FIG. 4

FIG. 5

FIG. 6

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **WANG et al.** Non-invasive Assessment of Hepatic Venous Pressure Gradient (HVPG) Based on MR Flow Imaging and Computational Fluid Dynamics. *Fluid Dynamics*, 21 September 2021, 33-42